# FASCICULE DE BREVET EUROPEEN

(11) **EP 0 769 754 B1**
(45) Date de publication et mention de la délivrance du brevet: **24.05.2000**
(21) Numéro de dépôt: 96402200.8
(22) Date de dépôt: 15.10.1996
(51) Int. Cl.: G06K 9/20, A61B 5/117, H01L 29/84, G01L 9/00

(54) **Capteur monolithique d'empreintes digitales**
Monolitischer Fingerabdrucksensor
Monolithic fingerprint sensor

(30) Priorité: 17.10.1995 FR 9512168
(43) Date de publication de la demande: 23.04.1997
(73) Titulaire: FRANCE TELECOM, 75015 Paris (FR); LA POSTE, 92777 Boulogne Billancourt Cédex (FR)
(72) Inventeur: Abou Hassan, Salman, Cabinet Ballot Schmit, 94230 Cachan (FR); Revillet, Marie-Josèphe, Cabinet Ballot Schmit, 94230 Cachan (FR)
(74) Mandataire: Schmit, Christian Norbert Marie

(56) Documents cités:
- EP-A- 0 457 398
- EP-A- 0 566 336
- FR-A- 2 674 051
- US-A- 4 393 773
- US-A- 5 438 875

## Description

L'invention concerne le domaine de l'authentification des personnes par le biais de la reconnaissance de leurs empreintes digitales.

On sait que les empreintes digitales sont utilisées depuis longtemps, en raison du lien pratiquement biunivoque qui existe entre une empreinte et une personne, c'est-à-dire que deux personnes différentes ont en principe des empreintes digitales différentes.

La méthode traditionnelle pour utiliser cette particularité de la nature est une méthode d'impression à base d'encre. Elle consiste à appuyer le doigt sur une surface encrée et reporter ce doigt sur une surface de papier blanche. Les lignes de crête appuient sur la surface blanche plus que les vallées; l'encre des lignes de crête se reporte mieux sur le papier que celle des vallées, et un dessin des lignes de crête apparaît nettement sur la surface blanche.

Cette méthode traditionnelle n'est pas agréable pour la personne qui doit encrer son doigt et le nettoyer après; et elle ne permet pas directement un traitement automatique de reconnaissance d'image.

D'autres méthodes ont été imaginées, à partir de systèmes optiques et électroniques, pour effectuer d'une part une prise d'image de l'empreinte et d'autre part un traitement par ordinateur de cette empreinte.

Ces méthodes impliquent en général un équipement important et des coûts élevés. Les méthodes optiques souffrent également de la sensibilité aux salissures, ce qui affecte la qualité du signal et introduit une baisse sensible de la fiabilité du système d'acquisition et d'identification.

Elles sont d'un usage peu commode du fait de l'entretien régulier qu'elles exigent.

D'autres systèmes de saisie d'empreinte existent, basés sur la transformation de l'empreinte digitale en un signal électrique qui, après mise en forme, peut être traité par un ordinateur.

Le brevet US 4 577 345 décrit un procédé et un dispositif de saisie et d'identification de l'empreinte utilisant un réseau de points de contact sur une puce de circuit intégré.

Des contacts électriques ponctuels peuvent être établis à chaque point du réseau par simple pression au dessus de ce point.

Les lignes de crête des empreintes digitales exercent une pression plus élevée que les vallées, et par conséquent, la pression du doigt peut établir un motif de contacts électriques correspondant aux lignes de crête, alors que les vallées définissent un motif sans points de contact.

Le motif ainsi dessiné peut être détecté par un réseau de transistors reliés aux points de contact possibles.

Dans la pratique, une membrane conductrice souple est tendue au dessus de la surface supérieure du circuit intégré et collée sur toute la périphérie de celui-ci. C'est elle qui établit les contacts électriques en chaque point du réseau en fonction du motif de pression exercé par le doigt.

Ce type de solution présente plusieurs inconvénients, parmi lesquels notamment le fait que le processus de fabrication est hétérogène puisqu'on mélange des technologies de circuit intégré et d'autres technologies.

L'assemblage de la membrane en particulier est une opération qui n'appartient pas à la technologie des circuits intégrés et qui est délicate à réaliser. Le coût de production reste donc élevé.

Par ailleurs, l'utilisation d'une membrane fragilise le système d'acquisition de l'empreinte. La membrane peut facilement se casser après quelques utilisations. Enfin, les membranes actuelles ne possèdent pas la finesse de grain requise pour une résolution suffisante permettant un traitement adéquat de l'image de l'empreinte.

L'invention a pour objet un capteur d'empreinte digitale qui ne présente pas les inconvénients rencontrés dans la technique antérieure.

Selon l'invention, on propose un capteur d'empreinte digitale qui comprend un moyen de détection de pression sensible au motif de l'empreinte et des circuits électroniques couplés à ce moyen de détection et permettant de fournir des signaux électriques représentant ce motif. Ce capteur est monolithique (sans pièce rapportée) et réalisé entièrement selon une technologie de fabrication de circuit intégré électronique aussi bien pour le moyen de détection de pression que pour les circuits électroniques couplés à ce moyen.

Les technologies de fabrication de circuit-intégré sont des techniques de dépôt en couches minces, photolithographie et gravure sèche ou humide de ces couches, dopage éventuel par des impuretés, et traitements thermiques divers. Elles sont effectuées par lots sur des tranches comportant plusieurs circuits-intégrés identiques.

Le capteur d'empreinte est donc constitué par un circuit intégré unique qui regroupe toutes les fonctions nécessaires pour transformer les reliefs de la peau du doigt (crêtes et vallées) en signaux électriques qui de préférence sont émis sous forme numérique (suite de signaux binaires). Il n'y a pas de membrane rapportée sur le circuit par un procédé de fixation mécanique; tous les éléments du capteur sont réalisés par dépôts et gravure de couches minces.

Le composant selon l'invention est de préférence réalisé selon un processus CMOS standard et utilisant les techniques de micro-usinage actuellement bien maîtrisées. Aucun assemblage de pièces réalisées séparément n'est nécessaire.

Le capteur d'empreinte constituera le point d'entrée d'un système d'identification et/ou d'authentification d'empreintes implanté sur un ordinateur personnel (PC) ou sur une station de travail raccordée à un ordinateur central.

Le système procède à une reconstitution de l'image de l'empreinte en utilisant les coordonnées (X,Y) de chaque point du motif relevé par le moyen de détection de pression.

L'ordinateur fournit au capteur des signaux de commande tels qu'une horloge, un signal de réinitialisation ("reset"). Le capteur étant en principe matriciel, le micro-ordinateur peut contrôler l'accès point par point aux différents points de la matrice. Les points de la matrice sont des microcapteurs de pression, l'ensemble des microcapteurs fournissant une image matricielle globale du motif de pressions résultant de l'application du doigt sur le capteur.

L'ordinateur reçoit du capteur un signal de mesure représentant la pression en chaque point, et les corrèle avec des signaux de comptage représentant la position de ce point dans une trame d'image.

L'image établie peut être traitée par un algorithme spécialisé et comparée à d'autres images stockées dans une base de données.

L'invention a donc pour objet un capteur d'empreinte digitale comprenant une matrice de microcapteurs de pression (12) sensible au motif de l'empreinte d'un doigt le capteur étant réalisé sur un substrat selon une technologie de fabrication de circuit intégré, principalement caractérisé en ce que chaque microcapteur de la matrice comprend une cavité (44) fermée par un diaphragme (46) constitué d'un matériau isolant pour porter des éléments conducteurs du microcapteur et permettre leurs déformations sous l'action d'une pression.

Elle a également pour objet un procédé de réalisation d'un capteur d'empreinte digitale comprenant une matrice de microcapteurs de détection de pression (12) sensible au motif de l'empreinte d'un doigt et des circuits électroniques (14, 16, 18, 20) couplés à ce moyen de détection et permettant de fournir des signaux électriques représentant ce motif, le capteur étant réalisé entièrement selon une technologie de fabrication de circuit intégré aussi bien pour la matrice de microcapteurs que pour les circuits électroniques couplés à la matrice caractérisé en ce qu'il comprend les étapes suivantes :
- dépôt d'une couche de silicium polycristallin (35) sur le substrat (30);
- photogravure de cette couche sauf dans les endroits de chaque microcapteurs;
- dépôt d'une couche d'isolant (36) sur toute la surface du substrat;
- dépôt d'une deuxième couche de silicium polycristallin (38);
- gravure de cette deuxième couche de silicium polycristallin (38) pour réaliser des ponts de résistances piézoélectriques correspondant chacun à un microcapteur;
- dépôt d'une deuxième couche d'isolant (40) pour protéger les résistances;
- attaque de la première couche de silicium polycristallin aux endroits non protégés formant ainsi une cavité sous les résistances piézoélectrique de manière à être suspendues à la couche isolante formée par les couches de nitrure de silicium (36, 40)

Elle a aussi pour objet un procédé de réalisation d'un capteur d'empreinte digitale comprenant une matrice de microcapteurs de détection de pression (12) sensible au motif de l'empreinte d'un doigt et des circuits électroniques (14, 16, 18, 20) couplés à ce moyen de détection et permettant de fournir des signaux électriques représentant ce motif, le capteur étant réalisé entièrement selon une technologie de fabrication de circuit intégré aussi bien pour la matrice de microcapteurs que pour les circuits électroniques couplés à la matrice caractérisé en ce qu'il comprend les étapes suivantes :
- dépôt d'une couche de métal et gravure pour former une première armature (51) de microcontacteur,
- dépôt d'une couche de silicium polycristallin (35) ou de métal;
- photogravure de cette couche pour obtenir un diaphragme de géométrie désirée;
- dépôt d'une couche de matériau isolant (36) sur toute la surface du substrat;
- dépôt d'une deuxième couche de métal et gravure pour former une deuxième armature (52) de microcontacteur;
- dépôt d'une couche d'isolant pour protéger les armatures;
- attaque de la couche de silicium polycristallin aux endroits non protégés formant ainsi une cavité à l'intérieur de laquelle se trouvent les armatures en vis à vis.

Selon un premier mode de réalisation les microcapteurs de pression fonctionnent selon un effet piézorésistif : ce sont des résistances dont la valeur varie avec la pression exercée.

D'autres effets physiques sont également envisagés pour détecter la pression en chaque point de la matrice. Selon un deuxième mode de réalisation chaque microcapteur de pression est constitué par un microcontacteur.

Selon un troisième mode de réalisation chaque élément microcapteur de pression est constitué par une capacité variable.

Dans le cas de résistances piézoélectriques, les microcapteurs sont formés d'un pont de résistances piézo déposées sur un diaphragme tendu au-dessus d'une cavité. La déformation du diaphragme, sous l'effet de la pression, provoque la déformation des résistances et donc une variation de leurs valeurs.

Dans le cas de microcontacteurs ou de condensateurs à capacité variable, le microcapteur est constitué de deux couches de métal distantes formant soit les armatures du condensateur à capacité variable, soit les bornes du microcontacteur, et une couche d'isolant (36) tendue au-dessus d'une cavité et formant un diaphragme, l'armature supérieure étant solidaire de ce diaphragme.

Le capteur selon la présente invention procure les avantages suivants par rapport aux solutions connues :
- meilleures précision,
- meilleure tenue mécanique,
- fabrication simplifiée (car il n'y a pas de problème d'assemblage d'une membrane ou de matériau piézo-électrique) donc coûts plus faibles,
- insensibilité à la température, par rapport à une technologie utilisant un matériau piézo-électrique, d'où une meilleure fiabilité.

D'autres caractéristiques et avantages de l'invention apparaîtront à la lecture de la description détaillée qui suit et qui est faite en référence aux dessins annexés dans lesquels :
- la figure 1 représente une vue générale du capteur ;
- la figure 2 représente la configuration électrique générale du capteur ;
- la figure 3 représente un diagramme de signaux électriques ;
- la figure 4 représente une coupe de la structure d'un microcapteur individuel ;
- la figure 5 représente un procédé de fabrication de cette première structure ;
- et la figure 6 représente un procédé de fabrication d'une deuxième structure conforme à l'invention.

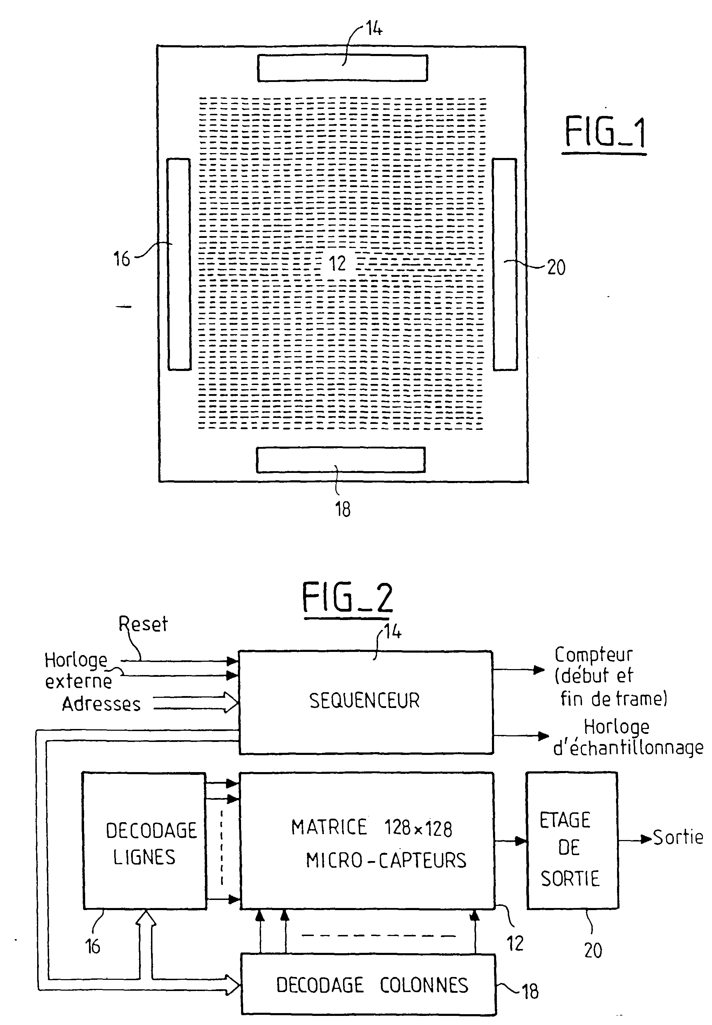

Sur la figure 1, on voit la constitution générale d'un capteur selon l'invention. Il comporte essentiellement une matrice de 128x128 éléments individuels qui sont des microcapteurs de pression disposés en réseau de lignes et colonnes.

Chaque élément occupe une surface carrée d'environ 50 micromètres par 50 micromètres, ou un cercle de 50 micromètres de diamètre environ.

Le pas du réseau est de préférence d'au plus 65 micromètres, c'est-à-dire que les centres de deux éléments adjacents sont séparés de 65 micromètres au plus : la surface globale de la matrice est de 8,3x8,3 millimètres carrés, suffisante pour saisir la partie centrale d'une empreinte digitale, qui est la zone la plus riche en informations. Les dimensions données ci-dessus correspondent à un exemple de réalisation; elles donnent une définition suffisamment précise de l'empreinte et sont réalisables technologiquement sans problème particulier.

Les microcapteurs peuvent être réalisés selon plusieurs techniques, utilisant des effets physiques différents pour la détection de pression. Les capteurs peuvent fonctionner sur un effet capacitif, ou un effet piézoélectrique, ou un simple microcontacteur intégré dans le circuit monolithique.

Chaque élément peut contenir une électronique simple permettant, par comparaison avec un seuil, de transformer la variation de pression en niveau logique binaire.

Dans le cas d'un microcapteur à effet capacitif, l'électronique de détection des variations de pression est cependant plus complexe et ne peut pas véritablement être incorporée dans chaque microcapteur; elle doit plutôt être installée à la périphérie du capteur. On peut prévoir éventuellement d'associer un point mémoire à chaque microcapteur à l'intérieur de la matrice.

Dans le cas d'un microcapteur à microcontacteur, l'électronique de détection est la plus simple à réaliser. L'information binaire est fabriquée dans le microcapteur même (l'interrupteur fermé ou ouvert selon la présence ou l'absence d'une pression).

Dans les deux cas de microcapteurs à effet capacitif et piézorésistif, il est possible d'obtenir un signal analogique. Cette possibilité permet d'effecteur des traitements plus élaborés sur l'image de l'empreinte (affichage en plusieurs niveaux de gris, traitement par réseaux de neurones, etc...).

La zone couverte de microcapteurs est désignée par la référence 12 au centre du circuit intégré monolithique. La périphérie est entourée de différents circuits qui sont notamment :
- un séquenceur 14, assurant le déroulement de la saisie d'information au moment de l'application du doigt. Le séquenceur comporte au moins un compteur (14 bits de préférence), un générateur de signaux d'horloge, et un sélecteur d'adresses ;
- un décodeur d'adresses de ligne 16;
- un décodeur d'adresses de colonne 18;
- un étage de sortie 20 qui comporte des circuits électroniques de détection et de traitement de signal analogique, dans le cas où les microcapteurs individuels ne comportent pas cette fonction, et des amplificateurs de sortie, pour transmettre à la sortie du capteur des informations représentant le motif de pression détecté par le réseau matriciel de microcapteurs.

La figure 2 représente le schéma électrique du capteur d'empreintes digitales. Le séquenceur 14 reçoit le signal de réinitialisation (reset), une horloge externe, et éventuellement des adresses pour l'exploration point par point ou ligne par ligne de l'image matricielle recueillie par les microcapteurs ; l'image peut cependant être transmise à l'extérieur du circuit intégré par un balayage systématique ligne par ligne analogue à un balayage d'image vidéo, le séquenceur comportant alors les éléments de circuit nécessaires pour réaliser ce balayage.

Le séquenceur transmet les adresses de ligne et de colonne aux décodeurs de ligne et de colonne (16 et 18) qui permettent d'accéder point par point aux microcapteurs de pression de la matrice 12. Le signal électrique en provenance d'un microcapteur adressé par ces décodeurs est transmis sur une sortie du capteur monolithique, à travers l'étage de sortie 20.

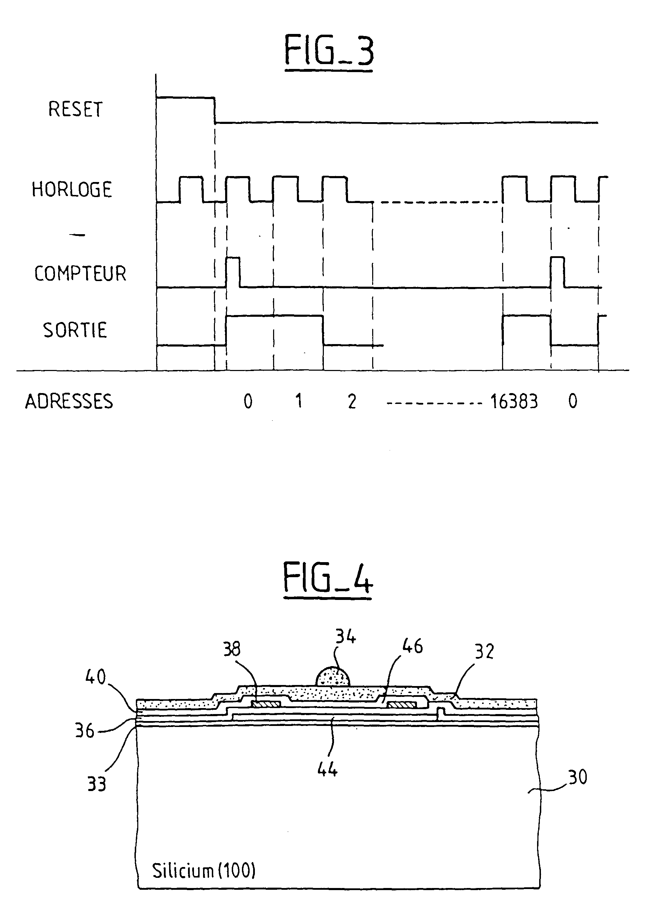

La figure 3 représente les signaux de sortie du capteur, et les signaux de commande associés. Le signal de réinitialisation (Reset) définit le début d'une exploration systématique de la matrice.

L'horloge définit le rythme d'exploration (par exemple un coup d'horloge pour chaque point de la matrice) ; les adresses successives sont incrémentées, par exemple ligne par ligne ; pour une matrice 128x128 il y a 16384 adresses successives à explorer. Le compteur de trame fournit une impulsion de remise à zéro pour définir chaque nouveau départ de transmission d'une image complète.

Les signaux de sortie transmis, dans le cas de résistances piézo, sont des signaux binaires représentant le dépassement ou le non dépassement d'un seuil de pression en chaque point de la matrice. Les signaux peuvent être transmis en série ou en parallèle par mots de 8 bits, 16 bits ou plus.

Dans le cas de microcontacteurs ou de condensateurs à capacité variable, le signal de sortie est analogique.

Lors d'une saisie d'empreinte digitale, le capteur entre en contact direct avec un milieu hostile. Il faut donc le protéger. Outre une couche de passivation qui termine le procédé de fabrication des circuits CMOS, on peut déposer une couche souple (de silicone par exemple) en protection finale.

Cette couche peut être utilisée également pour améliorer la fonction de transfert de pression entre le doigt et les microcapteurs. Dans ce cas, on peut réaliser dans cette couche un plot en surépaisseur au centre de chaque microcapteur, également pour améliorer cette fonction de transfert.

La figure 4 représente une coupe d'une telle structure avec une couche souple de protection 32 déposée sur la surface d'un substrat de circuit intégré 30 dans lequel ont été préalablement réalisés les microcapteurs de pression et toute l'électronique de traitement de signal correspondante.

La couche 32 présente, au centre de chaque microcapteur, une surépaisseur formant un plot central 34. La surépaisseur peut être obtenue par dépôt successif de deux couches, la première étant uniforme et la deuxième étant déposée à travers un masque délimitant les plots en surépaisseur.

Le matériau de la couche 32 est de préférence du silicone, à la fois souple et résistant.

Plusieurs techniques permettent de faire un capteur monolithique comportant des microcapteurs en silicium micro-usiné, compatibles avec la réalisation de circuits électroniques intégrés (de préférence en technologie CMOS).

L'exemple qui suit donne une réalisation détaillée pour des microcapteurs de pression utilisant un effet piézorésistif. Le procédé est compatible avec une technologie CMOS et permet donc l'intégration sur la même puce de circuit intégré des microcapteurs et des circuits électroniques de commande et de traitement de signal.

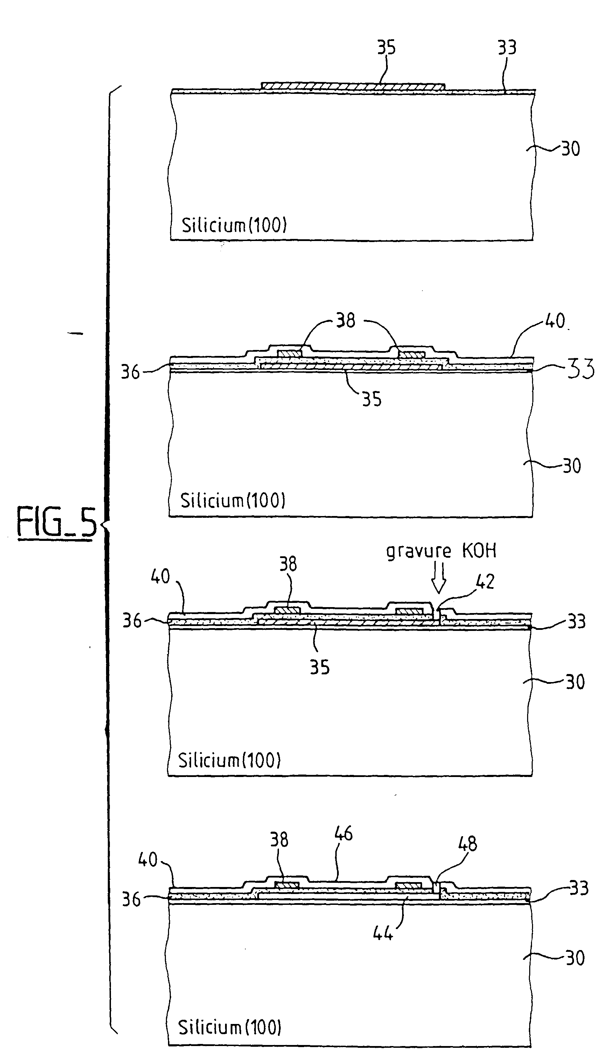

La figure 5 donne les principales étapes de réalisation.

Sur un substrat de silicium 30 éventuellement revêtu d'une fine couche d'arrêt de gravure 33 (en nitrure de silicium), on dépose une couche sacrificielle ou couche sacrifiée de silicium polycristallin 35.

Cette couche est photogravée pour laisser subsister une zone de silicium polycristallin à l'endroit de chaque microcapteur.

On dépose ensuite une fine couche de nitrure de silicium 36 sur toute la surface du substrat, puis une deuxième couche de silicium polycristallin 38. Cette deuxième couche est gravée pour réaliser des ponts de résistances piézoélectriques (un pont de Weathstone de 4 résistances) correspondant chacun à un microcapteur de pression.

Une ou plusieurs résistances d'un pont sont déposées au-dessus des zones de silicium polycristallin de la première couche. Puis, une deuxième couche de nitrure de silicium 40 est déposée pour protéger les résistances.

L'étape suivante consiste à pratiquer des trous 42 dans les couches de nitrure de silicium, à côté des résistances mais au dessus des zones de la première couche de silicium polycristallin 35.

Par ces trous on accède à la première couche de silicium polycristallin 35, à travers les deux couches de nitrure 36 et 40.

Une gravure humide (KOH) est réalisée pour attaquer le silicium polycristallin partout où il n'est pas protégé. L'attaque se fait par les trous 42 et la totalité de la couche sacrificielle 35 est enlevée à travers ces trous. Le silicium est protégé ailleurs par les couches de nitrure et par la couche d'arrêt de gravure 33.

Une cavité 44 subsiste alors sous les résistances piézoélectriques qui sont alors suspendues sur une couche isolante tendue et qui peuvent subir des déformations lorsqu'une pression est exercée sur elles.

La couche isolante tendue est ici constituée par les couches de nitrure de silicium 36 et 40 formant un diaphragme 46 suspendu au dessus de la cavité 44. Les parois latérales de la cavité sont en nitrure de silicium. Le fond est constitué par la fine couche d'arrêt de gravure 33 reposant sur le substrat.

La gravure terminée, on peut redéposer du nitrure de silicium 48 pour boucher les trous 42 et sceller ainsi les cavités.

Les circuits électroniques de commande et de traitement sont formés simultanément par des opérations de dépôt et de gravure classiques en technologie CMOS, certaines des étapes ci-dessus étant communes avec des étapes de réalisation de circuits CMOS, et toutes les étapes étant de toutes façons compatibles avec la réalisation de circuits CMOS sur le même substrat.

La couche souple 32 de protection (figure 4) en silicone ou matériau analogue est déposée en fin de procédé, après les étapes de passivation finale (couches de silice ou autres) classiques dans les circuits intégrés.

L'exemple qui suit donne une réalisation détaillée des microcapteurs de pression utilisant un effet capacitif ou des microcontacteurs. Dans les deux cas le processus de réalisation est le même, seule l'épaisseur du diaphragme change pour permettre ou non l'établissement d'un contact. Un ordre de grandeur de cette épaisseur est de 0,5 µm.

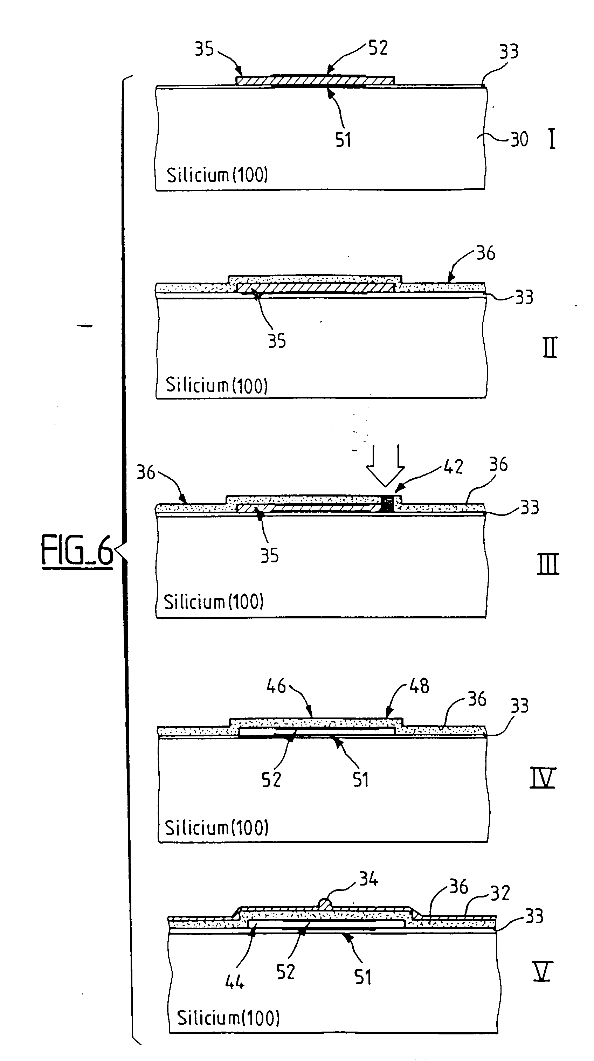

Dans cet exemple de réalisation illustré par la figure 6, on a repris les même références pour désigner les mêmes éléments que ceux entrant dans la description de la figure 5.

Sur un substrat de silicium on dépose une couche de métal (par exemple Au ou Ti) qui sera utilisée soit comme première armature 51 d'une capacité variable, soit comme première borne d'un microcontacteur.

Cette couche 51 est gravée pour former la géométrie (circulaire dans cette réalisation) et l'emplacement de l'armature.

On dépose ensuite une couche intermédiaire de silicium polycristallin 35 ou une couche de métal tel que de l'aluminium (Al) (couche sacrifiée). Cette couche 35 subit une photolithogravure pour obtenir la géométrie (circulaire) et l'emplacement du diaphragme 46 du microcapteur.

Le diaphragme 46 et l'armature 52 sont concentriques. Une deuxième couche de métal 52 (Au ou Ti) est déposée pour former soit la deuxième armature de la capacité, soit la deuxième borne du microcontacteur. Cette couche est gravée pour laisser subsister la deuxième armature dans l'alignement vertical de la première.

La deuxième étape consiste à déposer une couche fine de nitrure de silicium 36 sur toute la surface du substrat.

La troisième étape consiste à pratiquer un trou sur le bord du diaphragme 36 et qui traverse la couche de nitrure pour atteindre la couche sacrifiée. Ensuite, une gravure humide est effectuée sur le substrat. La solution attaque à travers le trou la couche sacrifiée. La gravure continue jusqu'à disparition totale de la couche sacrifiée. Une cavité est donc formée. Le diaphragme 46 et l'armature supérieure 52 se trouvent complètement dégagés.

La gravure étant terminée, on effectue un dépôt de nitrure de silicium 48 sous vide pour fermer le trou et sceller la cavité (bouchon 48).

La dernière étape consiste à déposer une couche de silicone 32 pour protéger l'ensemble et réaliser un plot centré au milieu de chaque microcapteur (étape V).

## Revendications

1. Capteur d'empreinte digitale comprenant une matrice de microcapteurs de pression (12) sensible au motif de l'empreinte d'un doigt le capteur étant réalisé sur un substrat selon une technologie de fabrication de circuit intégré, caractérisé en ce que chaque microcapteur de la matrice comprend une cavité (44) fermée par un diaphragme (46) constitué d'un matériau isolant pour porter des éléments conducteurs du microcapteur et permettre leurs déformations sous l'action d'une pression.

2. Capteur d'empreinte selon la revendication 1 , caractérisé en ce que chaque microcapteur de pression est constitué par un pont de résistances piézoélectriques (38) déposé sur une première couche de matériau isolant (36) formant le diaphragme.

3. Capteur d'empreinte selon la revendication 2, caractérisé en ce que le pont de résistances piézoélectriques est constitué par des bandes de silicium polycristallin (38) déposée sur la première couche isolante (36 ) tendue au dessus de la cavité (44).

4. Capteur d'empreinte selon la revendication 3, caractérisé en ce que la cavité est délimitée par des parois formées par cette première couche isolante, les résistances piézoélectriques reposant sur le diaphragme (46) suspendu au dessus de la cavité.

5. Capteur d'empreinte selon la revendication 1 , caractérisé en ce que chaque microcapteur de pression est constitué par un microcontacteur dont les armatures sont dans la cavité, l'une étant solidaire du diaphragme (46) , l'autre étant sur le substrat (30).

6. Capteur d'empreinte selon la revendication 1, caractérisé en ce que chaque microcapteur de pression est constitué par une capacité variable dont les armatures sont dans la cavité , l'une étant solidaire du diaphragme, l'autre étant sur le substrat.

7. Capteur d'empreinte selon les revendications 5 ou 6, caractérisé en ce que la cavité est délimitée par des parois en nitrure de silicium (36), une couche de métal (52) reposant sur le diaphragme (46) suspendu au-dessus de la cavité.

8. Capteur d'empreinte selon l'une des revendications précédentes, caractérisé en ce que la matrice comporte au moins 128X128 microcapteurs agencés selon un réseau de pas inférieur ou égal à 65 micromètres.

9. Capteur selon l'une quelconque des revendications précédentes, caractérisé en ce que les microcapteurs sont réalisés sur un substrat monolithique et sont revêtus d'une couche (32) en matériau souple tel que du silicone protégeant les microcapteurs et facilitant la fonction de transfert de pression entre le doigt et les microcapteurs.

10. Capteur selon la revendication 9, caractérisé en ce que l'épaisseur de la couche de matériau souple est renforcée au centre de chaque microcapteur pour former un plot (34) et améliorer ainsi la fonction de transfert de pression entre le doigt et les microcapteurs.

11. Capteur d'empreinte selon l'une quelconque des revendications précédentes, caractérisé en ce qu'il comprend des circuits électroniques comportant des moyens (20) pour transmettre sur une sortie du capteur des signaux électriques représentant la pression détectée par chaque microcapteur.

12. Capteur d'empreinte selon la revendication 11, caractérisé en ce que les signaux électriques représentant la pression détectée par un microcapteur sont des signaux binaires représentant le dépassement ou l'absence de dépassement d'un seuil de pression.

13. Capteur d'empreinte selon l'une quelconque des revendications précédentes, caractérisé en ce que le signal de sortie est analogique permettant une mesure plus précise de la pression du doigt.

14. Procédé de réalisation d'un capteur d'empreinte digitale comprenant une matrice de microcapteurs de détection de pression (12) sensible au motif de l'empreinte d'un doigt et des circuits électroniques (14, 16, 18, 20) couplés à ce moyen de détection et permettant de fournir des signaux électriques représentant ce motif, le capteur étant réalisé entièrement selon une technologie de fabrication de circuit intégré aussi bien pour la matrice de microcapteurs que pour les circuits électroniques couplés à la matrice caractérisé en ce qu'il comprend les étapes suivantes :
- dépôt d'une couche de silicium polycristallin (35) sur le substrat (30);
- photogravure de cette couche sauf dans les endroits de chaque microcapteurs;
- dépôt d'une couche d'isolant (36) sur toute la surface du substrat;
- dépôt d'une deuxième couche de silicium polycristallin (38);
- gravure de cette deuxième couche de silicium polycristallin (38) pour réaliser des ponts de résistances piézoélectriques correspondant chacun à un microcapteur;
- dépôt d'une deuxième couche d'isolant (40) pour protéger les résistances;
- attaque de la première couche de silicium polycristallin aux endroits non protégés formant ainsi une cavité sous les résistances piézoélectrique de manière à être suspendues à la couche isolante formée par les couches de nitrure de silicium (36, 40)

15. Procédé de réalisation d'un capteur d'empreinte digitale comprenant une matrice de microcapteurs de détection de pression (12) sensible au motif de l'empreinte d'un doigt et des circuits électroniques (14, 16, 18, 20) couplés à ce moyen de détection et permettant de fournir des signaux électriques représentant ce motif, le capteur étant réalisé entièrement selon une technologie de fabrication de circuit intégré aussi bien pour la matrice de microcapteurs que pour les circuits électroniques couplés à la matrice caractérisé en ce qu'il comprend les étapes suivantes :
- dépôt d'une couche de métal et gravure pour former une première armature (51) de microcontacteur,
- dépôt d'une couche de silicium polycristallin (35) ou de métal;
- photogravure de cette couche pour obtenir un diaphragme de géométrie désirée;
- dépôt d'une couche de matériau isolant (36) sur toute la surface du substrat;
- dépôt d'une deuxième couche de métal et gravure pour former une deuxième armature (52) de microcontacteur;
- dépôt d'une couche d'isolant pour protéger les armatures;
- attaque de la couche de silicium polycristallin aux endroits non protégés formant ainsi une cavité à l'intérieur de laquelle se trouvent les armatures en vis à vis.

16. Procédé selon les revendications 14 ou 15, caractérisé en ce que les couches d'isolant sont des couches de nitrure de silicium.

## Patentansprüche

1. Monolithischer Fingerabdrucksensor mit einer Matrix (12) aus Mikrodrucksensoren, empfindlich für das Abdruckmuster eines Fingers, wobei der Sensor auf einem Substrat gemäß einer IC-Herstellungstechnik realisiert wird,
**dadurch gekennzeichnet,**
dass jeder Mikrosensor der Matrix einen Hohlraum (44) enthält, verschlossen mittels einer durch ein Isoliermaterial gebildeten Membran (46), die die leitfähigen Elemente des Mikrosensors trägt und ihre Verformungen unter der Wirkung eines Drucks zulässt.

2. Abdrucksensor nach Anspruch 1, dadurch gekennzeichnet, dass jeder Mikrodrucksensor durch eine piezoelektrische Widerstandsmessbrücke (38) gebildet wird, die auf einer ersten Schicht aus Isoliermaterial (36) abgeschieden ist, die die Membran bildet.

3. Abdrucksensor nach Anspruch 2, dadurch gekennzeichnet, dass die piezoelektrische Widerstandsmessbrücke durch Streifen aus polykristallinem Silicum (38) gebildet wird, abgeschieden auf der ersten, über den Hohlraum (44) gespannten Isolierschicht (36).

4. Abdrucksensor nach Anspruch 3, dadurch gekennzeichnet, dass der Hohlraum durch Wände abgegrenzt wird, die durch diese erste Isolierschicht gebildet werden, wobei die piezoelektrischen Widerstände auf der Membran (46) ruhen, die über den Hohlraum gespannt ist.

5. Abdrucksensor nach Anspruch 1, dadurch gekennzeichnet, dass jeder Mikrodrucksensor durch einen Mikroschalter gebildet wird, dessen Belegungen sich in dem Hohlraum befinden, wobei die eine mit der Membran (46) verbunden ist und die andere mit dem Substrat (30).

6. Abdrucksensor nach Anspruch 1, dadurch gekennzeichnet, dass jeder Mikrodrucksensor durch eine variable Kapazität gebildet wird, deren Belegungen sich in dem Hohlraum befinden, wobei die eine mit der Membran (46) verbunden ist und die andere mit dem Substrat.

7. Abdrucksensor nach einem der vorangehenden Ansprüche, dadurch gekennzeichnet, dass der Hohlraum durch Wände aus Siliciumnitrid (36) abgegrenzt wird, wobei eine Metallschicht (52), die auf der Membran (46) ruht, über dem Hohlraum hängt.

8. Abdrucksensor nach einem der vorangehenden Ansprüche, dadurch gekennzeichnet, dass die Matrix wenigstens 128x128 Mikrosensoren umfasst, gitterförmig angeordnet mit einer Teilung kleiner oder gleich 65 µm.

9. Abdrucksensor nach einem der vorangehenden Ansprüche, dadurch gekennzeichnet, dass die Mikrosensoren auf einem monolithischen Substrat realisiert und mit einer Schicht (32) aus nachgiebigem Material wie z.B. Silikon überzogen sind, das die Mikrosensoren schützt und die Funktion der Druckübertragung zwischen dem Finger und den Mikrosensoren erleichtert.

10. Abdrucksensor nach Anspruch 9, dadurch gekennzeichnet, dass die Dicke der Schicht aus nachgiebigem Material in der Mitte jedes Mikrosensors verstärkt wird, um einen Knopf (34) zu bilden und so die Funktion der Druckübertragung zwischen dem Finger und den Mikrosensoren zu verbessern.

11. Abdrucksensor nach einem der vorangehenden Ansprüche, dadurch gekennzeichnet, dass er elektronische Schaltungen umfasst, die Einrichtungen (20) enthalten, um über einen Ausgang des Sensors elektrische Signale zu übertragen, die den durch jeden Mikrosensor detektierten Druck repräsentieren.

12. Abdrucksensor nach Anspruch 11, dadurch gekennzeichnet, dass die den durch einen Mikrosensor detektierten Druck repräsentierenden elektrischen Signale Binärsignale sind, die das Überschreiten oder das Nichtüberschreiten einer Druckschwelle darstellen.

13. Abdrucksensor nach einem der vorangehenden Ansprüche, dadurch gekennzeichnet, dass das Ausgangssignal analog ist, was eine genauere Messung des Fingerdrucks ermöglicht.

14. Verfahren zur Herstellung eines Fingerabdrucksensors mit einer für das Abdruckmuster eines Fingers empfindlichen Matrix (12) aus Mikro-Druckdetektionssensoren und elektronischen Schaltungen (14, 16, 18, 20), gekoppelt mit diesen Detektionseinrichtungen, die dieses Muster darstellende elektrische Signale liefern, wobei der Sensor ganz gemäß einer IC-Herstellungstechnik realisiert wird, sowohl bezüglich der Mikrosensoren-Matrix als auch bezüglich der mit der Matrix gekoppelten elektronischen Schaltungen,
**dadurch gekennzeichnet**, dass es die folgenden Schritte umfasst:
- Abscheiden einer Schicht aus polykristallinem Silicium (35) auf dem Substrat (30) ;
- Photoätzen dieser Schicht mit Ausnahme der Mikrosensorenstellen;
- Abscheiden einer Isolierschicht (36) auf der gesamten Oberfläche des Substrats:
- Abscheiden einer zweiten Schicht aus polykristallinem Silicium (38);
- Ätzen dieser zweiten Schicht aus polykristallinem Silicium (38), um piezoelektrische Widerstandsmessbrücken herzustellen, von denen jede einem Mikrosensor entspricht;
- Abscheiden einer zweiten Isolierschicht (40), um die Widerstände zu schützen;
- Angreifen der ersten Schicht aus polykristallinem Silicium an den ungeschützten Stellen, um so unter den piezoelektrischen Widerständen einen Hohlraum zu bilden, so dass sie in der durch die Siliciumnitridschichten (36, 40) gebildeten Isolierschicht aufgehängt sind.

15. Verfahren zu Herstellung eines Fingerabdrucksensors mit einer für das Abdruckmuster eines Fingers empfindlichen Matrix aus Mikro-Druckdetektionssensoren (12) und elektronischen Schaltungen (14, 16, 18, 20), die mit diesen Detektionseinrichtungen gekoppelt sind und elektrische Signale liefern, die diese Muster darstellen, wobei der Sensor ganz gemäß einer IC-Herstellungstechnik realisiert wird, sowohl bezüglich der Mikrosensoren als auch bezüglich der mit der Matrix gekoppelten Schaltungen,
**dadurch gekennzeichnet**, dass es die folgenden Schritte umfasst:
- Abscheiden und Ätzen einer Metallschicht, um eine erste Mikroschalter-Belegung (51) zu bilden,
- Abscheiden einer Schicht aus polykristallinem Silicium (35) oder aus Metall;
- Photoätzen dieser Schicht, um eine Membran der gewünschten Geometrie zu erhalten;
- Abscheiden einer Schicht aus Isoliermaterial (36) auf der gesamten Oberfläche des Substrats;
- Abscheiden und Ätzen einer zweiten Metallschicht, um eine zweite Mikroschalter-Belegung (52) zu bilden;
- Abscheiden einer Isolierschicht, um die Belegungen zu schützen;
- Angreifen der Schicht aus polykristallinem Silicium an den ungeschützten Stellen, um so einen Hohlraum zu bilden, in dessen Innern sich die Belegungen gegenüberstehen.

16. Verfahren nach den Ansprüchen 14 oder 15, dadurch gekennzeichnet, dass die Isolierschichten Siliciumnitridschichten sind.

## Claims

1. Fingerprint sensor comprising a matrix of pressure microsensors (12) sensitive to the pattern of the print of a finger, the sensor being produced on a substrate according to an integrated circuit manufacturing technology, characterised in that each microsensor of the matrix comprises a cavity (44) closed by a diaphragm (46) constituted by an insulating material to carry conducting elements of the microsensor and allow their deformations under the action of a pressure.

2. Fingerprint sensor according to claim 1,
characterised in that each pressure microsensor is constituted by a bridge of piezo-electric resistors (38) deposited on a first layer of insulating material (36) forming the diaphragm.

3. Fingerprint sensor according to claim 2,
characterised in that the bridge of piezo-electric resistors is constituted by strips of polycrystalline silicon (38) deposited on the first insulating layer (36) stretched over the cavity (44).

4. Fingerprint sensor according to claim 3,
characterised in that the cavity is delimited by walls formed by this first insulating layer, the piezo-electric resistors resting on the diaphragm (46) suspended over the cavity.

5. Fingerprint sensor according to claim 1,
characterised in that each pressure microsensor is constituted by a microswitch the armatures of which are in the cavity, one being locked to the diaphragm (46), the other being on the substrate (30).

6. Fingerprint sensor according to claim 1,
characterised in that each pressure microsensor is constituted by a variable capacitor the armatures of which are in the cavity, one being locked to the diaphragm, the other being on the substrate.

7. Fingerprint sensor according to claims 5 or 6,
characterised in that the cavity is delimited by walls made of silicon nitride (36), a layer of metal (52) resting on the diaphragm (46) suspended over the cavity.

8. Fingerprint sensor according to one of the preceding claims, characterised in that the matrix comprises at least 128X128 microsensors arranged in a network with a pitch less than or equal to 65 micrometres.

9. Sensor according to any one of the preceding claims,
characterised in that the microsensors are produced on a monolithic substrate and are covered with a layer (32) of flexible material such as silicone protecting the microsensors and facilitating the pressure transfer function between the finger and the microsensors.

10. Sensor according to claim 9, characterised in that the thickness of the layer of flexible material is reinforced in the centre of each microsensor to form a contact (34) and thus improve the pressure transfer function between the finger and the microsensors.

11. Fingerprint sensor according to any one of the preceding claims, characterised in that it comprises electronic circuits comprising means (20) for transmitting electrical signals representing the pressure detected by each microsensor at an output of the sensor.

12. Fingerprint sensor according to claim 11,
characterised in that the electrical signals representing the pressure detected by a microsensor are binary signals representing the overshooting or non-overshooting of a pressure threshold.

13. Fingerprint sensor according to any one of the preceding claims, characterised in that the output signal is analogue permitting more precise measurement of the pressure of the finger.

14. Process for production of a fingerprint sensor comprising a matrix of pressure-detecting microsensors (12) sensitive to the pattern of the print of a finger and electronic circuits (14, 16, 18, 20) coupled to this means of detection and making it possible to supply electrical signals representing this pattern, the sensor being produced entirely according to an integrated circuit manufacturing technology both for the matrix of microsensors and for the electronic circuits coupled to the matrix, characterised in that it comprises the following steps:
- deposition of a layer of polycrystalline silicon (35) over the substrate (30) ;
- photo-engraving of this layer except in the locations of each microsensor;
- deposition of a layer of insulating material (36) over the entire surface of the substrate;
- deposition of a second layer of polycrystalline silicon (38);
- engraving of the second layer of polycrystalline silicon (38) to produce bridges of piezo-electric resistors each corresponding to a microsensor;
- deposition of a second layer of insulating material (40) to protect the resistors;
- attacking the first layer of polycrystalline silicon in the unprotected locations thus forming a cavity under the piezo-electric resistors so as to be suspended from the insulating layer formed by the layers of silicon nitride (36, 40).

15. Process for producing a fingerprint sensor comprising a matrix of pressure-detecting microsensors (12) sensitive to the pattern of the print of a finger and electronic circuits (14, 16, 18, 20) coupled to this means of detection and making it possible to supply electrical signals representing this pattern, the sensor being produced according to an integrated circuit manufacturing technology both for the matrix of microsensors and for the electronic circuits coupled to the matrix, characterised in that it comprises the following steps:
- deposition of a layer of metal and engraving to form a first microswitch armature (51);
- deposition of a layer of polycrystalline silicon (35) or of metal;
- photo-engraving of this layer to obtain a diaphragm of desired geometry;
- deposition of a layer of insulating material (36) over the entire surface of the substrate;
- deposition of a second layer of metal and engraving to form a second microswitch armature (52);
- deposition of a layer of insulating material to protect the armatures;
- attacking the layer of polycrystalline silicon in the unprotected locations thus forming a cavity inside which the armatures are located facing one another.

16. Process according to claims 14 or 15, characterised in that the layers of insulating material are layers of silicon nitride.
